# EUROPEAN PATENT APPLICATION

(11) **EP 2 369 516 A2**
(43) Date of publication of application: **28.09.2011**
(21) Application number: 11165184.0
(22) Date of filing: 24.09.2002
(51) Int. Cl.: G06F 19/00, A61J 1/14

(54) **Methods and apparatuses for assuring quality and safety of drug administration and medical products and kits**

(30) Priority: 24.09.2001 US 324043 P
(62) Divisional of application: 02768888.6
(71) Applicant: Scott Laboratories, Inc., Lubbock, TX 79415 (US)
(72) Inventor: Hickle Randall S., Lubbock, TX 74910 (US); Cobb Nicholas Edward, Lubbock, TX 79415 (US)
(74) Representative: Mozzi, Matteo

(57) **Abstract**

The present invention provides apparatuses and methods for marking components, supplies and kits of drug administration devices and other medical systems with quality assurance information. The invention also provides apparatuses and methods for tracking time of use of such components, supplies and kits and various apparatuses and methods for preventing use or reuse of tainted, recalled or unrecognized components, supplies and kits. Quality assurance markers (QAMs) are described which store information regarding the identity and manufacturer of disposable components, supplies and kits. The invention utilizes several QAM modalities, such as, among others, 1-D and 2-D bar codes, 1-D and 2-D symbologies, holograms, written text, radio frequency identification devices (RFIDs), integrated chip smart cards, and EEPROMs.

## Description

### FIELD OF THE INVENTION

The present invention relates generally to systems and methods for encoding information related to medical supplies, components, and kits and detecting, among other things, use and reuse of those items so as to assure quality and safety of those items.

### BACKGROUND OF THE INVENTION

Many medical procedures, such as the administration of drugs (e.g., sedative and analgesic drugs) are safety-critical tasks with patient health at issue. The margin for error in administration of such drugs may be small because of the narrow range between a correct dose and an overdose. Patient harm and even death may be consequences of this small margin for error. Therefore, identification and certification of the origin and manufacturer of medical components, supplies, kits and the like, and identification of drugs to be administered to a patient is important. This identification and certification enhances patient safety by ensuring the quality of pharmaceuticals and enhances the quality of single-patient use disposable products by assuring such criteria as sterility, calibration, and manufacturing tolerance.

Also important are means for preventing tainted medical components, supplies and kits already used for the administration of drugs to one patient from being subsequently reused with another patient. Cross-contamination between patients is a concern because of infectious diseases caused by blood-borne pathogens such as the Human Immunodeficiency Virus (HIV) and hepatitis B and C and by respiratory pathogens such as multi-drug resistant tuberculosis. Infection with any of these pathogens can be lethal and reuse of tainted medical supplies, components and kits among different patients has an attendant risk of such infection. Further, contamination of certain pharmaceuticals has caused fatal cases of septicemia because these compounds support the growth of bacteria.

In an attempt to prevent cross-contamination, tainted medical equipment, components and supplies are often sterilized prior to reuse with a different patient. However, recent studies indicate that sterilization of many medical products, especially, for example, those that have valves, complex mechanisms, or narrow and long lumens (e.g., laparoscopic trocars, endoscopic biopsy forceps, and fiberscopes), may not be entirely effective.

An alternative way to avoid cross-contamination is through single patient use (disposable) medical components and supplies. Disposable medical components and supplies generally do not prevent cross-contamination if they are reused. Therefore, concerns remain as to both the deliberate and the unintentional reuse of tainted disposable medical components and supplies. There is also concern beyond patient cross-contamination with the unauthorized sterilization and/or reuse of disposable medical components and supplies that are not designed or validated to be sterilized or to have a long service or product life.

Goods that outwardly and superficially look like a component or supply of a medical device or system having the appropriate form, fit, and function to be used with the device or system may actually be uncertified products that were otherwise not manufactured according to original design specifications. In many circumstances where quality and reliability of performance are mission-critical, customers or other users may not be able to discern the difference between uncertified and genuine components, supplies, parts and kits. For example, proper use of a drug administration or infusion device or system requires knowledge of, for example, the drug concentration, dead space or internal volume in the drug delivery tubing and drug pump cassette, and calibrated tubing and compression surfaces (in order to generate accurate volumetric control of the rate of drug delivery). This information may not be known or be outside the specifications for uncertified versions of components, supplies and kits of drug delivery devices and systems.

If medical components, supplies and kits were "smart," the detection of the presence or absence of certified medical components, supplies and kits and their use-condition could be automated. Automation may also relieve clinicians of the chore and memory load of certifying products and may enhance patient safety by ensuring that all necessary components, supplies and kits are present and where appropriate, unused, before the initiation of a medical procedure.

Further, despite the best quality assurance efforts of manufacturers, contaminated or defective products sometimes reach the marketplace. Ensuing product recalls or other measures taken to address defective products can be a costly endeavor for manufacturers. A tagging identification system that would facilitate localization and removal of potentially every single recalled product would be advantageous. As an added safety measure, it would be beneficial if the batch number and unique identifiers of the recalled products could be programmed into associated delivery devices or systems, such as sedation and analgesia machines, or at dispensing locations like pharmacies, stock or supply rooms or a centralized database so that any product, such as a tainted drug vial slipping through a recall, could be identified and rejected.

A smart card or chip card can comprise at least one integrated circuit chip (memory and/or microprocessor) which may be encased in a piece of plastic. The plastic may be of similar format to a credit card. Data transfer from the integrated circuit chip to a reader can be via gold plated contacts or without contacts (such as, for example, via radio frequencies used in RFID chips). Some smart cards may also incorporate magnetic stripes and/or barcodes as optional features. Smart cards may be classified as memory only, stored value, debit, or microprocessor based. Multifunction smart cards make use of spare processing and/or storage capacity on smart cards to implement additional functions such as identification and storage of health data. The function of a smart card derives mainly from its IC and the data and firmware stored in the IC.

### SUMMARY OF THE INVENTION

The present invention provides solutions to the above quality and safety concerns by presenting, for example, various devices and methods for marking the disposable components, supplies and kits of drug administration devices and systems with quality assurance information, various devices and methods for tracking time since potential exposure to airborne organisms, and various devices and methods for preventing use or reuse of tainted, recalled or unrecognized components, supplies and kits.

The invention may be applicable to stand-alone drug delivery devices such as, among others, a stand-alone or autonomous infusion pump as well as drug delivery systems that may be integrated with patient health monitors and other subsystems. The invention is also broadly applicable to medical devices and systems that use disposable and certain reusable medical components, supplies and kits.

Information regarding the identity and manufacturer of disposable components, supplies and kits and the drug to be administered may be encoded in quality assurance modules or markers (QAMs) provided with the components, supplies and kits. Various marker modalities, such as, among others, 1-D and 2-D bar codes, 1-D and 2-D symbologies, holograms, written text, radio frequency identification devices (RFIDs), integrated chip smart cards, and EEPROM type markers, may be used. The information encoded on QAMs may be encrypted for security and it may include an identification means unique to each individual tagged medical component, supply or part. All such information is detected by a reader device (which may be a reader, scanner, or imaging engine, among other devices) and is presented to an electronic controller of a drug administration device or system for evaluation and/or comparison to product data and safety data stored in memory or accessible by the electronic controller. The interpretation or translation of data encoded in the markers may be performed at a reader, at a controller of the drug administration device or system or at a remote site operably connected to a controller of a drug administration device or system. Only when information encoded in a product's QAM is indicated to be valid to a controller, will the product be accepted for use with the device or system.

To prevent unsafe reuse of tainted disposable components, supplies and kits of a drug administration device or system, the present invention presents systems for the detection and refusal of used components, supplies and kits. Unused components, supplies and kits are modified upon their first use with a drug administration device or system. Detectors are presented which sense the presence or absence of such modification. An electronic controller of the device or system will only accept a component or supply and allow administration with it attached to the device or system if a signal from the reuse detectors indicates the absence of a use-modification.

According to a further embodiment, the invention comprises a medical product marked with a quality assurance module, wherein said quality assurance module stores information relating to the product, said information comprising an identifier unique to the medical product and/or to the origin of the product.

According to a further embodiment, said medical product is selected from the group consisting of a drug administration drug container, a drug administration cassette, and a drug administration kit.

According to a further embodiment said medical product is a drug container, and said information further comprises information relating to properties of the drug container and the drug provided therein.

According to a further embodiment, said information further comprises information selected from the group consisting of the concentration of the drug provided in the container, the drug mix, identification of the manufacturer of the drug and/or the container, an expiration date of the drug and/or the container, the dimensions of the container, the nominal drug volume when the container is full of drug, a network address.

According to a further embodiment, said medical product is a drug cassette, and said information further comprises information relating to properties of the drug cassette.

According to a further embodiment, said information further comprises information selected from the group consisting of identification of the manufacturer of the cassette, a product ID for the cassette, a lot number for the cassette, an expiration date for the cassette, the internal or dead space volume of drug flow channels associated with the cassette, and a value reflecting the flow resistance of drug flow channels associated with the cassette.

According to a further embodiment, a drug container having a quality assurance module storing information relating to the container is coupled to the cassette, and wherein the quality assurance module of the cassette is positioned such that quality assurance modules of both the container and the cassette are within a common scan field of a reader device which senses the information stored on the modules.

In accordance with a further embodiment, the invention comprises an integrated drug delivery system, which comprises: a patient health monitor device adapted so as to be coupled to a patient and generate a signal reflecting at least one physiological condition of the patient; a drug delivery controller supplying one or more drugs to the patient; at least one medical product removably coupled to the system, said product having a quality assurance module for storing information regarding the product, wherein the quality assurance module comprises an identifier unique to the medical product and/or to the origin of the product; a reader device for reading information stored on the quality assurance module of said medical product; a memory device storing a safety data set reflecting safe and undesirable parameters of at least one monitored patient physiological condition and reflecting safe and undesirable parameters of the medical product; and an electronic controller interconnected between the patient health monitor, the drug delivery controller, the reader device, and the memory device storing the safety data set; wherein said electronic controller receives said signals and in response manages the application of the drugs in accord with the safety data set.

According to a further embodiment, the invention comprises a drug delivery apparatus, said apparatus comprising: a drug delivery controller supplying one or more drugs to the patient; and at least one medical product removably coupled to the apparatus, said product having a quality assurance module for storing information regarding the product, wherein the quality assurance module comprises an identifier unique to the medical product and/or to the origin of the product.

### BRIEF DESCRIPTION OF THE DRAWINGS

**FIG. 1** is a schematic diagram of a particular embodiment of the present invention.
**FIG. 2** is an elevation view of one embodiment of the invention in which QAMs are positioned within a common reading field.
**FIG. 3** is an elevation view of one embodiment of the present invention in which QAMs are positioned within a common and vertical reading field.
**FIG. 4** is an elevation view of one embodiment of the present invention in which one product has a 1 D bar code QAM and the other product has a 2D bar code QAM both of which are positioned within a common reading field.
**FIG. 5** is an elevation view of one embodiment of the present invention in which the vial QAM is a radio frequency identification tag and the cassette QAM is a bar code.
**FIG. 6** is an elevation view of one embodiment of the present invention in which the vial QAM and the cassette QAM are both holograms.
**FIG. 7** is an elevation view of one embodiment of a cassette reuse indication device according to the present invention.
**FIG. 8** is an elevation view of an alternative embodiment of a cassette reuse indication means according to the present invention.

### DETAILED DESCRIPTION OF THE INVENTION

The present invention comprises apparatuses and methods that enhance quality assurance, prevent misuse, and facilitate product recall, tracking, or similar measures taken with respect to medical products. This description of the invention will focus on those apparatuses, devices and methods applicable to drug administration, but they are broadly applicable to other medical products and kits in other medical fields such as, among others, dialysis cartridges and kits, and diagnostic kits.

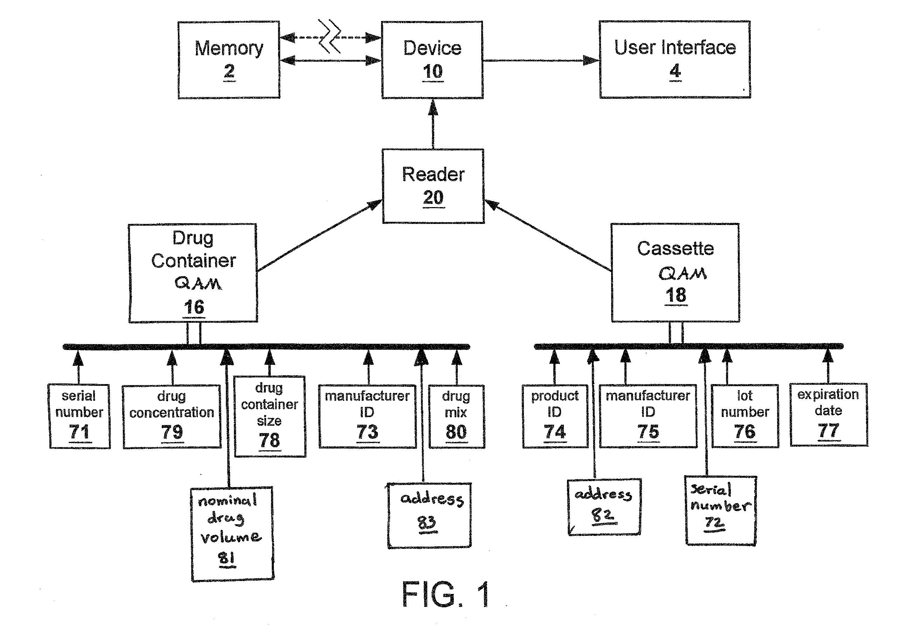

**FIG. 1** shows the flow of data between the various elements of one embodiment of the present invention. Quality assurance modules or markers (QAMs) are provided with disposable components, supplies, and kits of components or supplies of drug administration device or system 10. Preferably, QAMs encode information as to the source and identification of the component, supply, or kit, or in the case of a drug container, information as to the drug itself.

An example of drug administration device or system **10** is described in U.S. Patent Application Serial No. 09/324,759, filed June 3, 1999 and incorporated herein by reference. The sedation and analgesia system of Application No. 09/324,759 includes a patient health monitor device adapted so as to be coupled to a patient and generate a signal reflecting at least one physiological condition of the patient, a drug delivery controller supplying one or more drugs to the patient, a memory device storing a safety data set reflecting safe and undesirable parameters of at least one monitored patient physiological condition, and an electronic controller interconnected between the patient health monitor, the drug delivery controller, and the memory device storing the safety data set; wherein said electronic controller receives said signals and in response manages the application of the drugs in accord with the safety data set. The safety data set, as referred to by the electronic controller, may further include data regarding proper values for the identification and/or sources of drugs, supplies, components or kits.

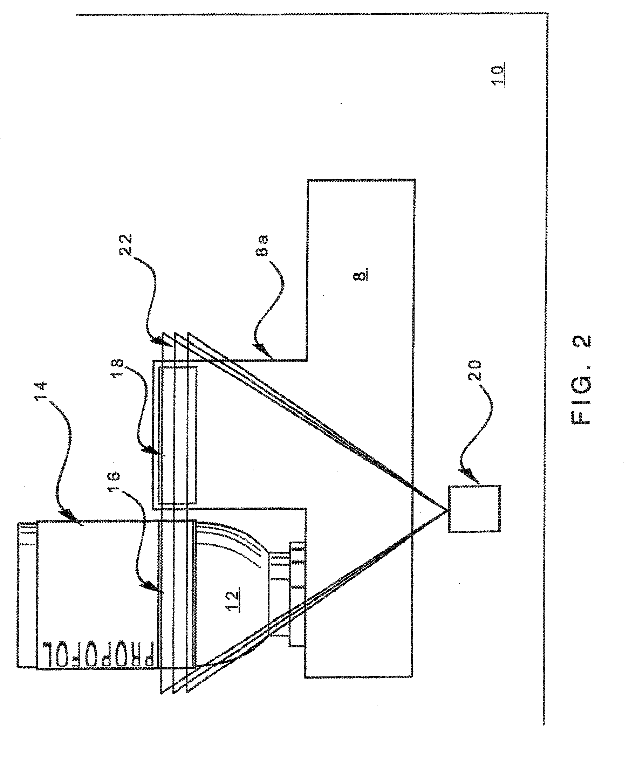

Examples of components or supplies that may be used with device or system **10** and marked with QAMs include drug container **12** and drug cassette **8** (shown in **FIG. 2**). Drug container **12** may be marked with drug container QAM **16**, such as, among others, a tag or label, that identifies its contents by providing certain information relevant to users of device or system **10**. Drug cassette 8, may be marked with cassette QAM **18**. An example of drug cassette **8** is described in U.S. Patent Application Serial No. 10/208,184, filed July 31, 2002, and incorporated herein by reference in its entirety. Disposable kits, supplies or other components of device or system **10** may also be marked in the manner in which cassette 8 and container **12** are marked in this description.

Still referring to **FIG. 1**, the information encoded in drug container QAM **16** may include, among other things, drug container size, volume, or cross-sectional area **78**; drug concentration **79**; drug mix **80** (e.g., a local anesthetic and Propofol or an opioid and Propofol); unique and/or proprietary serial number **71**; manufacturer ID **73**; nominal drug volume when the container is full **81**; and address **83**, such as a Uniform Resource Locator (URL) also known as a Uniform Resource Identifier(URI), where up to date information, such as, among others, recall information, labeling recommendations, prescribing recommendations, contra-indications, potential drug interactions, and adverse drug reactions may be accessed via an electronic network.

The information encoded in a cassette QAM **18** may include, among other things, product ID **74**; manufacturer ID **75**; lot number **76**; unique serial number **72**; and expiration date **77**. Cassette QAM **18** may comprise both high and low data versions of the information encoded. A high data version of cassette QAM **18** may encode manufacturer identification **75**, product identification **74**, lot number **76**, address **82**, such as a Uniform Resource Locator (URL) also known as a Uniform Resource Identifier(URI), where up to date information may be accessed via an electronic network, and expiration date **77**. An electronic controller of administration device or system **10** uses product identification for drug cassettes to properly control priming and purging volumes and set occlusion detection thresholds by matching product ID **74** with known characteristics, such as the deadspace or internal volume and flow resistance, of the particular cassette. A low data version of cassette QAM **18** is also contemplated whereby the stored data may be a subset of the parameters encoded in the high data version.

Still referring to **FIG. 1**, the data encoded in QAMs **16** and **18** are read by a reader **20**. Reader **20** communicates the data to an electronic controller or CPU provided with a drug administration device or administration system **10**. Device or system **10** stores the data and references pre-programmed data, such as a certified manufacturer list, from resident (solid bi-directional arrow) or remote (phantom bi-directional arrow with break marks) memory **2**. Device or system **10** may also communicate the data received from reader **20** to users via a user interface **4**.

When a QAM stores an expiration date, such as expiration date **77** on cassette QAM **18**, and reader **20** communicates to the controller of delivery device or system **10** that the component, supply, or drug having the QAM is past the expiration date, the controller may prevent the use of the expired component, supply or drug.

When a QAM stores unique serial number **71, 72**, or a serial number assigned to some other disposable component, the serial number may be sensed by reader **20** and then archived in memory **2**. An electronic controller or CPU provided with device or system **10** then accesses the stored serial number prior to initiating use of the drug container or cassette or other component or supply. If the stored serial number read by reader **20** matches a serial number already archived in memory **2**, the electronic controller will reject the component or supply from use as having been previously used and tainted. Such rejection of previously used and/or tainted drug administration components, supplies and kits embodies a quality assurance aspect of the present invention by preventing the risk of cross-contamination attendant with the reuse of tainted disposable components, supplies and kits.

In an alternative embodiment of the invention, the data encoded in the QAMs and the data relevant to drug administration, including the unique serial numbers, may be stored in memory that is at a remote location, like a hospital information system (HIS) computer or data bank or a centralized regional, national or international data bank. A controller of drug administration device or system **10** is operably connected to such remote memory.

It is contemplated that components, supplies or kits having QAMs encoded with a unique serial number could be readily logged and inventoried by both manufacturers and users. A product recall (or any other process that could involve tracking products) of particular components, supplies and kits marked with unique serial numbers according to the present invention, would be facilitated by logs and inventories tied to those numbers because recalled products could be individually located and quickly separated from non-recalled products. It is also contemplated that recalled product information could be programmed, entered or downloaded and stored in resident or remote memory **2** of drug administration device or system **10**. The unique serial numbers and other product information such as batch numbers encoded in QAMs of components, supplies and kits presented by a user for use with device or system **10** may then be compared to the stored recall data by an electronic controller. If the products' QAM data matches its respective recall data, the controller may reject or prevent the particular component or supply from use in a drug administration run. With a database that comprises the unique serial numbers, it is also possible to trace each item which has been used and therefore not available for recall. Further, it is possible to trace each patient that received, was treated or was in contact with recalled products in the event that notification or follow up is required.

Identification information of components, supplies and kits may also be compared to pre-programmed manufacturer data stored in resident or remote memory **2** of device or system **10**. If the manufacturer information stored in a product's QAM does not match the pre-programmed information regarding certified manufacturers, an electronic controller may reject or prevent the product from use as having been manufactured by an uncertified entity. Such rejection of drug administration device or system components, supplies and kits without certified manufacturer identification embodies a quality assurance aspect of the present invention by preventing the risk to patient safety resulting from product incompatibility or poor product performance attendant with the use of disposable components, supplies and kits produced by unrecognized or uncertified entities.

Notification of any of the above rejections may be communicated to users of administration device or system **10** via a user interface **4**. In further embodiments, any one or all pieces of information encoded in a product's QAM may be communicated to users by any of various means including audible or visual devices. For example, the identity of a product's manufacturer encoded in the product's QAM may be audibly represented to users by, among others, a unique signature tone, sound, or sequence of sounds or visually by a unique image, logo, name, or acronym displayed on a screen of the user interface device.

In a preferred embodiment, a unique serial number or a portion of that number that is encoded in a product's QAM is encrypted. Merely by way of example, the serial number may be encrypted by adding an extra digit, for example, among others, at the end of the serial number where that extra digit is, for example, among others, the second integer of the quotient of the division of the serial number by, for example, among others, 7. For example, if the serial number is 16735894 (and the quotient of a division of that number by 7 is thereby 2390842), then the extra digit is 3 and the encrypted serial number would be 167358943. Upon the QAM being sensed, the marker reader or the electronic controller of the device or system or a remote site operably connected to an electronic controller of the device or system would register the unique serial number-extra digit combination, sever the last digit and perform a pre-programmed calculation to check whether the digit matched the encryption scheme. If there was no match, the reader or controller or remote site operably connected to the controller would reject the serial number as being uncertified. Using the above example, an encrypted serial number of 167358947 would be rejected. Various other encryption schemes are available and may be employed. For example, a serial number may be rejected if the encryption code read from a QAM does not equal the result of a modulo 26 (remainder after division of the serial number by 26) operation of the serial number (0-25) directly mapped to the letters A through Z, or alternatively "a" to "z". The decryption of the data encrypted in the markers may be performed at the reader, at the controller of the drug administration device or system or at a remote site operably connected to a controller of the drug administration device or system.

The serial number may be a character sequence of numbers, letters, or other symbols that have an identifiable uniqueness such that a mechanism which assigns the serial number to each component, supply or kit and places the serialized QAM on the component, supply or kit can keep track of each serial number used. For example, an eight digit number may be used for the serial number and this mechanism can assign each marked component or supply with a unique number between 00,000,000 and 99,999,999 inclusive yielding 100 million unique serial numbers. As described above, an additional ninth character may be added at any location within the string of digits forming the serial number as an encryption scheme. Additionally, the amount of unique serial numbers available can be upgraded by allocating additional digits and/or alphanumeric characters (including lower case and upper case) to the serial number, for example, increasing the number of digits allocated to the unique serial number from 8 to 10 and 12 and 14 and so on as demand for the products grows and the original unique serial numbers start to be used up.

Referring again to **FIG. 1**, drug container size **78** may refer to container size or nominal volume of drug stored in a drug container and may be used to predict impending exhaustion of the drug in a given drug container during drug administration. The initial volume of drug may be known from the data stored in QAM **16**. The time when a given drug container is placed onto drug administration device or system **10** can be determined using reader **20**. It is the time when QAM **16** is first sensed. The time when drug flow is first initiated can be determined as the first time when flow through the drug delivery device such as, among others, an infusion pump is non-zero for a given drug container. By integrating the drug flow rate delivered by delivery device or system **10** (including drug flow for priming and purging) over time, the volume extracted from the given drug container can be inferred as well as the volume of drug remaining. The start time for the integration period could be, among others, any of: first detection of a new drug container, or first non-zero flow through the drug delivery device or system. The user can be notified of the impending exhaustion of the drug via user interface **4**. The controller may also shut down the delivery device or system **10** or place it in a heightened state of preparation for shutdown if impending exhaustion of the drug is indicated.

Any of the times (first detection of a new drug container, time of spiking, and first non-zero drug flow for a given drug container) or a combination of the times can be used by an electronic controller of device or system **10** to infer the time when the drug in a given drug container is potentially first exposed to airborne organisms. A timer provided with device or system **10** counts the elapsed time from that first instance of potential exposure to airborne organisms and notifies the controller when a particular time threshold, e.g., 6 hours for propofol, has elapsed. Upon such notification, the controller may then discontinue administration from the drug container and request insertion of a new drug container and cassette. When 6 hours have elapsed since infusion began for a given vial, the software may generate a "Vial Expired" system advisory alarm. When 6 hours have elapsed since infusion began for a given cassette, the software may display a "Cassette Expired" system advisory alarm. When 6 hours have elapsed for either a drug container or a cassette, the software may display a "Change Cassette and Vial" message.

**FIG. 2** shows a marker system according to an embodiment of the present invention in which drug container **12** and drug cassette **8** are marked with QAMs **16** and **18** respectively. Container QAM **16** is placed on an edge of existing manufacturer's label **14** or on container **12** itself. QAM **16** may be positioned and/or designed to not hide any printed information on existing label **14**. QAM **16** may be placed on label **14** either at the label manufacturer's facility or at the pharmaceutical facility that supplies the drug. As an illustrative example, a QAM or repeated series of QAMs may be placed along an edge of an existing label on, for example, a 20 ml Astra-Zeneca Diprivan™ propofol vial, which may include the manufacturer's own bar codes and other information, so as to not interfere with the existing printed information on the label.

Container QAM **16** may be repeated at regular or irregular intervals around a circumference or perimeter of existing label **14** or on container **12** itself. For example, identical copies of a particular type of QAM may be placed onto the surface of a generally cylindrical drug container every half inch around a circumference of the container. This embodiment ensures that reader **20** may remain fixed in place and still read container QAM **16** without users of the device or system **10** having to orient container **12** to be in a prime position to be read.

**FIG. 2** shows cassette QAM **18** on a flat surface of an extension **8a** of cassette **8** that is within the same scanning field **22** of QAM reader **20** as is container QAM **16**. Cassette QAM **18** may be placed on various surfaces of cassette **8**, including fin or extension **8a** which is constructed so as to be in a common scan field with drug container QAM 16 when container **12** is spiked onto cassette **8**.

When cassette **8** and drug container **12** both have QAMs encoding data according to the present invention, the QAM of each product must be readable. Preferably, a single reader **20** is positioned so as to be able to sense the QAMs **16** and **18** (and any other QAMs on associated products) from a fixed position. To accomplish this, cassette QAM **18** and container QAM **16** may be positioned on their respective products so as to be in a common zone or field of scan lines **22** when those products are inserted into position with administration device or system **10**. Therefore, the housing of reader **20** does not have to be repositioned after reading one QAM in order to read another associated QAM.

Alternatively, the QAMs may be placed such that they are not in a common zone or field that is readable by a single fixed reader necessitating the use of more than one reader or the use of complex mechanisms to redirect the scan field of a single reader such that it can read non-contiguous or non-aligned QAMs.

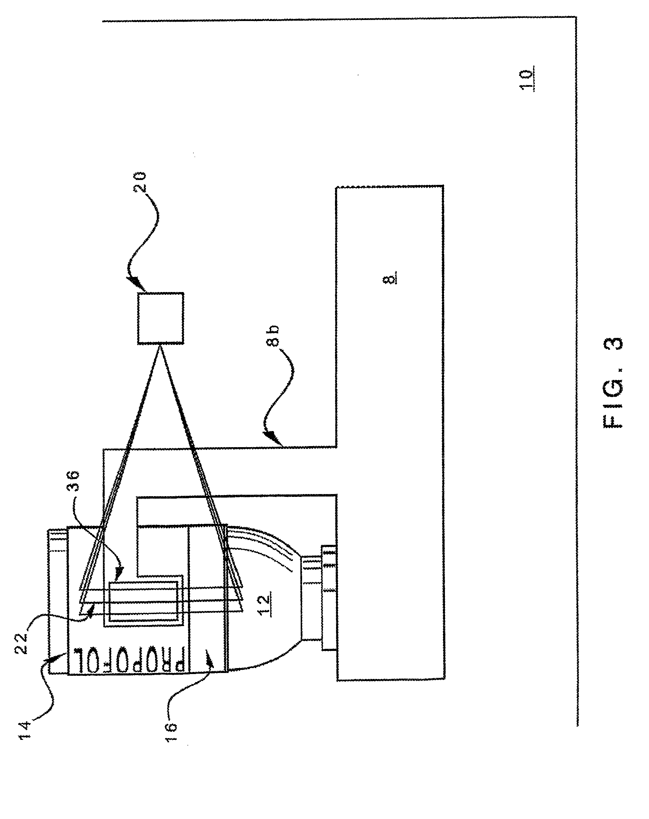

**FIG. 3** shows a marker system according to an alternative embodiment of the present invention in which container QAM **16** and cassette QAM **36** are positioned vertically with respect to drug container **12**. In this embodiment, drug container QAM **16** may still be repeated in a continuous ring around a circumference or perimeter of existing label **14** or of the container **12** itself, but the actual symbols of QAM **16** are read within vertical scan field **22** of reader **20**.

Various types of markers are contemplated for use with the present invention. For example, any one of or any combination of the following marker types, among others, may be used: bar codes, holograms, written text, radio frequency identification devices (RFIDs), and EEPROM type markers. The above list is not meant to be exclusive and other means of marking the above information on disposable components, supplies and kits of drug administration devices and systems may be used with the present invention.

Reader **20** may run software or other firmware and is capable of reading at least one, but preferably more than one type of marker provided with the present invention. For example, a bar code reader is provided to read bar code QAMs, an optical character recognition (OCR) device is provided to read written QAMs, a laser is provided to read hologram QAMs, and an RFID antenna and reader circuit is provided to read RFID QAMs. Detectors which sense the presence or absence of a feature that is modified upon first use, by detection of the unmodified feature prior to first use, can also be used to identify the presence of drug vials and disposable components, supplies and kits from manufacturers of certified quality.

It is contemplated that when more than one component or supply include QAMs that are each to be read, they may be read in combination by a single QAM reader. Scanners exist, and are contemplated for use with the present invention, that may read different types of QAM modalities (such as bar codes, holograms, written text, RFIDs, and EEPROM type devices). For example, U.S. Patent No. 6,264,106 describes a combination RFID-bar code scanner that is capable of sensing and interpreting information encoded in both an RFID device and a bar code. Therefore, each of various disposable components, supplies and kits of drug administration device or system **10** may be marked using a different QAM modality. A single component or supply may also possess more than one QAM or type of QAM modality, and a reader capable of sensing each may be employed.

Referring again to **FIG. 3**, in embodiments of the present invention in which drug container QAMs are bar codes, for example, maintaining a substantially zero degree angle of incidence may be less of an issue with low density codes scanned vertically (e.g., along the centerline of the vial) as it is with low density codes positioned and scanned horizontally around the circumference or perimeter of container **12**. This is because, relative to vertical scan field **22** of reader **20,** the surface of drug container **12** and hence QAM **16** does not curve away from the plane of scan field **22** for readers requiring line of sight. A vertical cassette QAM **36** is placed on cassette extension **8b** such that it also is within vertical scan field **22** of reader **20.**

In one embodiment, the QAM comprises a symbol such as a bar code. Various bar code symbologies exist and it is contemplated that any one or combination of more than one symbology may be used to mark disposable components, supplies and kits of drug administration devices and systems according to the present invention. Depending upon the symbology, the size of a bar code used to mark disposable components, supplies and kits can vary. Preferably also, when used on a curved surface like that of a vial, bar code symbols are sized or selected (such as high density symbologies of small size) so that the reader beam impinges onto the bar code at a substantially zero degree angle of incidence. The angle of incidence is defined as the angle between the incident reader beam and the normal to the bar code (an imaginary line perpendicular to the surface of the bar code). A substantially zero angle of incidence helps ensure that the reader beam remains a tight focused spot upon impinging the bar code, allowing both low and high density bar codes to be accurately read. As an extreme example, if the angle of incidence were to be close to 90 degrees (incident reader beam is almost parallel to bar code), the reader beam would be smeared into a large oval upon impinging the bar code and most of the reflected beam would be directed away from the reader, making it very difficult if not impossible to read a bar code. When placed on a curved surface such as that of a vial, as a bar code becomes wider and curves further away from the scan plane of the reader, the angle of incidence of the reader beam will become larger and the likelihood that the reader will be unable to read the bar code will increase. This problem may be applicable to all symbols such as barcodes and holograms on curved surfaces that need to be read by an electromagnetic beam such as laser and light beams and may be more pronounced with low density barcodes which tend to be wider. Low density bar codes may pose little distortion problems when placed on flat surfaces, such as may be provided on the surface of a drug cassette.

A preferred way of being able to size symbols such as bar codes to ensure a substantially zero degree angle of incidence of the reader beam on symbols affixed to curved surfaces is to select high density symbologies, which require less area of the surface they are provided upon than do low density symbologies in order to portray the same amount of encoded information. Certain embodiments of the present invention are contemplated, therefore, to use high density bar codes for the QAMs on cassettes, drug containers and associated drug administration products, to use a high density symbol for only drug container QAMs while using a low density symbol for cassette QAMs, or to use low density symbols for the QAMs on both the cassette and the drug container provided the low density symbol on a drug container that has a curved surface is capable of being sized so as to ensure a substantially zero degree angle of incidence of the reader beam. In an exemplary embodiment, a 2D bar code symbology, such as MaxiCode and/or Micro PDF417, is used as a high density QAM on a drug container while a 1 D bar code is used as a low density QAM on a drug cassette.

As noted above, it is contemplated that a combination of bar code symbologies, e.g., high and low density, may be used in combination for marking disposable components, supplies and kits according to the present invention. It is further contemplated that more than one bar code symbology may be used in combination within a single marker on a single disposable component or supply. Readers, scanners, and imaging engines exist, and are contemplated for use with the present invention, that are capable of reading more than one bar code symbology in combination. An example of such a scanner is the Symbol Technologies 2223 bar code scanner.

In embodiments of the present invention in which bar codes are used as the markers, the symbols of the code may be but need not be printed on labels or dedicated areas with visible ink. In order to preserve the aesthetics of the label or dedicated area, the bar codes or symbols may be printed with invisible inks, such as infrared or ultraviolet inks. An invisible bar code also presents an added security feature to the marking technology of the present invention in that it may not readily be detected and, therefore, may not be easily reproduced or modified without certification.

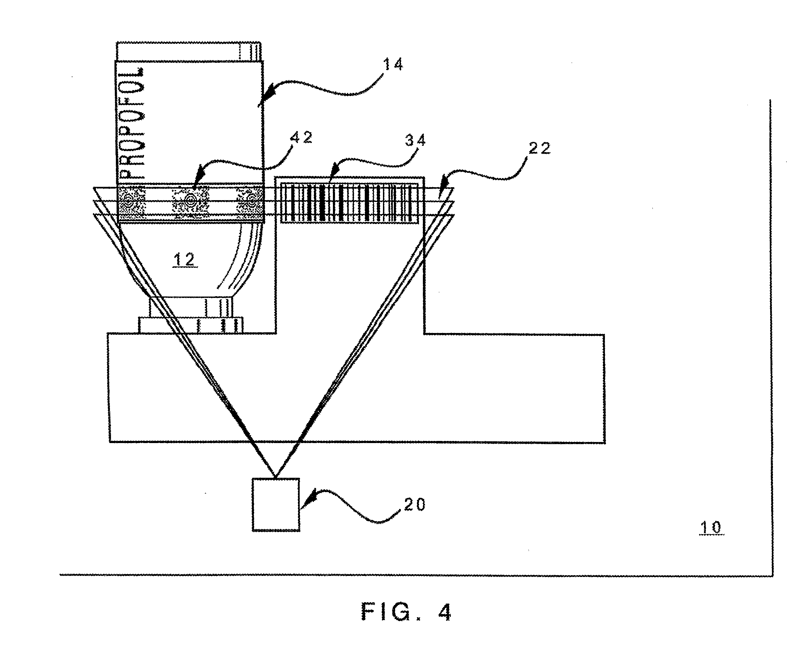

**FIG. 4** shows a marker system according to a further alternative embodiment of the present invention in which drug container QAM **42** is a high density 2D bar code while cassette QAM **34** is a low density 1 D bar code. In this embodiment, 2D bar code drug container QAM **42** is still placed in a continuous ring of repeating codes around a circumference or perimeter of existing label **14** or of the container **12.** Reader **20** may be capable of reading both high density drug container QAM **42** and low density cassette QAM **34** with the same scanning field **22.**

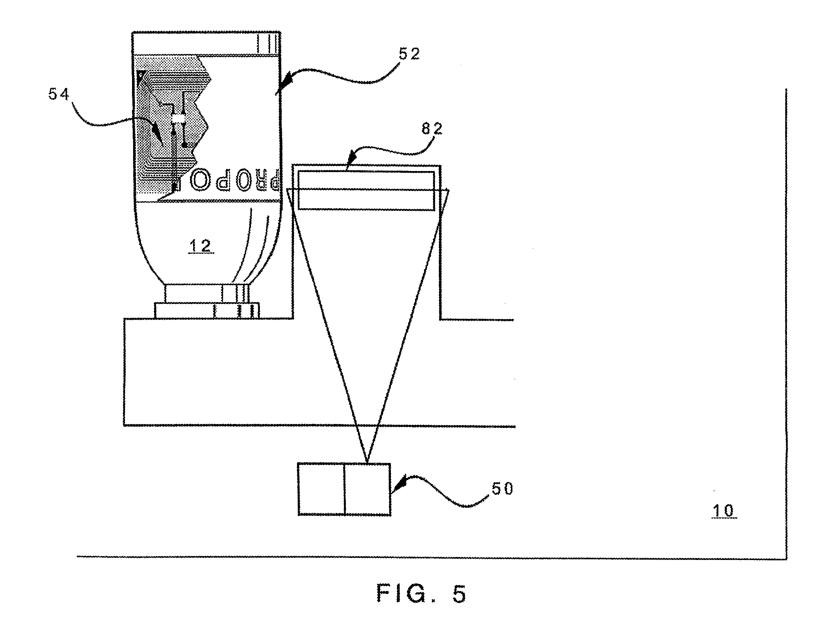

**FIG. 5** shows a marker system according to an even further alternative embodiment of the present invention in which drug container QAM **54** is a passive, inexpensive, disposable, non-contact, non-volatile read/write radio frequency identification ("RFID") tag embedded within a drug container label **52**. Cassette QAM **82** is shown as a generic bar code. A combined RFID-bar code reader and scanner **50** that is capable of sensing encoded information within both RFID tags and bar codes is also shown. The RFID marker may not necessarily be embedded in the label and may be visibly affixed to the container, label, component or supply. RFID devices may be embedded within special product labels wherein the labels may still be able to display the routine manufacturer applied information on their surfaces. When embedded, the RFID device may be hidden from view beneath the label's surface. The RFID device may also be applied to the surface of a product or an existing manufacturer's label on a disposable product for use with a drug administration device or system. In this embodiment, an RFID reader is employed to sense the information encoded within RFID QAMs.

Reader **50** could interface to an RFID QAM via, among other techniques, inductive coupling (e.g., by using an antenna) or capacitive coupling (e.g., by using conductive carbon ink that picks up electrostatic charges from reader). RFID tags from manufacturers like TI, Motorola, Philips, Mitsubishi, Intermec, Micron and SCS may be used as a QAM according to the present invention. In particular embodiments of the present invention, RFID QAM **54** is a small (e.g., 1.8" x 1.8"), thin (e.g., 0.015" maximum /0.003" minimum), self-adhesive label such as the inductively-coupled Tag-it RFID label from Texas Instruments which features 256 user programmable data bits at 4 feet read range and at 13.56 MHz. Such tags can be substituted for a regular label on a vial (e.g., a vial of Propofol or other drug or any other medical fluid) or other disposable or reusable medical supply or component. As will be appreciated by one skilled in the art, 256 bits can generate 1.2 x 10⁷⁷ (i.e., 2²⁵⁶) unique ID numbers.

RFID QAMs are autoclavable and resist dirt, grease, scratches, and wear and tear. More than one RFID QAM can be read from at the same time. RFID readers do not require line of sight reading nor direct contact with or relative movement to the tags. RFID readers can read at distances exceeding 4 feet depending on antenna size and power. Tampering with QAM **54** is discouraged by embedding the RFID tag within label **52** or within container **12** itself during manufacture, to make it inaccessible or whereby physical access to the imbedded tag would disable the medical supply, e.g., by creating a leak in an otherwise airtight system.

Electronic tags, such as Electrically Erasable Programmable Read Only Memory (EEPROM) integrated circuits ("IC"), (e.g., Microchip 24C00) may also be used as QAMs according to the present invention. Further examples of RFID QAMs and EEPROM QAMs are disclosed in U.S. Patent Application Serial No. 10/151,255, filed May 21, 2001 and incorporated herein by reference.

Alternatively, magnetic strips like those at the back of credit cards can be used as QAMs for medical supplies, components, and kits. When magnetic strips are used, a read head may be implemented to contact the magnetic strip when the strip is moved past the read head. This movement does not need to be constant but cannot be zero. This movement may be obtained when the read head is positioned on administration system or device **10** such that during the physical installation of a medical supply, component, or kit with system or device **10**, the magnetic strip QAM contacts the read head and moves relative to it. Similarly, upon removal of the medical supply, component, or kit from system or device **10** after its use, the magnetic strip may also contact a read/write head so that its encoded data is altered or its serial number is logged to indicate that the particular supply, component, or kit has been used.

Further, smart cards can be used as the QAMs for medical supplies, components, and kits. A smart card or integrated circuit (IC) chip QAM could be incorporated into the part, tethered to the part or physically separate from the part. A smart card QAM without contacts could be incorporated into a medical product in a similar manner to RFID QAMs and a smart card QAM with contacts could be incorporated into a medical product in a similar manner to EEPROM QAMs. This incorporation could be effected by embedding the IC chip into the material of the medical product.

A smart card QAM may also be tethered to a medical supply, component, and kit or to a group of those products tagged according to the present invention. The tethering means may be a plastic loop or any other such device. The tethering means may also be made of a suitable design and material in order to prevent accidental or deliberate severing or tampering. Smart card QAMs without contacts may be more suited for tethering as compared to a contact QAM because no manual aligning of the contact-less smart card would need to be made with the reader **20**. Alternatively, a smart card QAM may be separate from the medical product for which it encodes data. Separate smart card QAMs may be packaged along with the medical product. Separate smart card QAMs may also encode data for more than one medical product and could be packaged with the entire group, or a portion thereof, of those products for which the smart card QAM encodes data.

Other QAM implementations may be used according to the present invention, including physical indicators such as thermochromatic ink that changes color on heating or "scratch-and-sniff" coatings may be used in certain situations and on certain items to be tagged. Further, alternative electronic data transfer mechanisms, such as Bluetooth for example, could be used as QAMs.

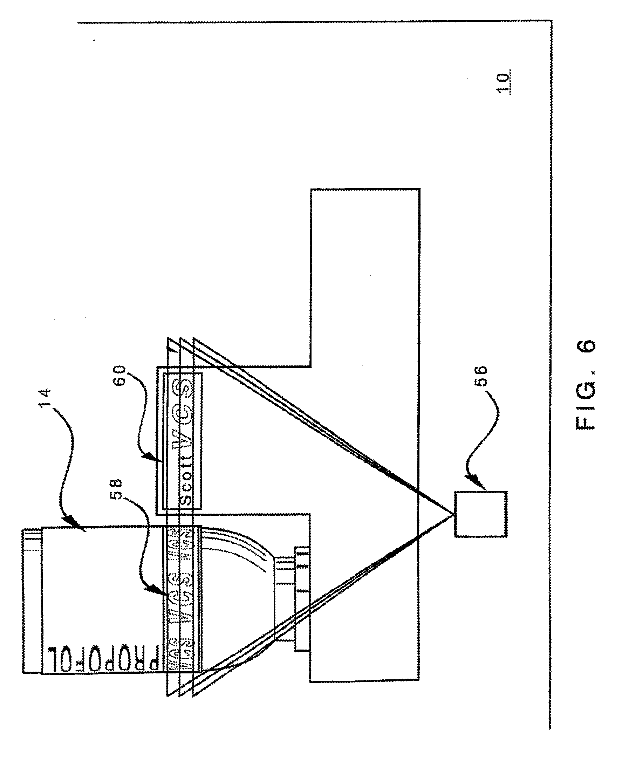

**FIG. 6** shows a marker system according to yet another alternative embodiment of the present invention in which drug container QAM **58** and cassette QAM **60** are both holograms. Hologram reader **56** is shown which can sense the information encoded on both product QAMs. The types of holograms that may be used as QAMs according to the present invention are various and include, but are not limited to: embossed, photopolymer, dichromated gelatin, silver halide, and/or optically variable devices (OVDs). The impression of the hologram may be embossed into a substrate material such as plastic film, metallized paper and/or transparent film. In embodiments in which the QAM is a photopolymer hologram, the hologram may be produced using a photo sensitive medium that is preferably deposited onto a polyester film substrate. In embodiments in which a hologram QAM is produced from dichromated gelatin, the substrate used is preferably glass. Glass substrates may be especially applicable to the glass walls of certain drug containers. In embodiments in which a hologram QAM is produced by a silver halide process, the photosensitive medium is deposited on polyester or acetate film substrate. In embodiments in which a hologram QAM is an OVD, the OVD may preferably be a diffractive optically variable image device (DOVID) or an optical foil or film product, but it may also be an ink product. OVDs provide extra quality assurance to the QAMs of the present invention in that they may be frustrating to would-be purveyors of uncertified components, supplies and kits. The image of a hologram QAM may be any one or combination of various different styles including 3D, 2D, 2D3D, uniform repeating pattern, diffraction grating, dot matrix, covert image, stereogram image, multi-channel image, white light transmission, and white light reflection. In this embodiment, a hologram reader may be employed to sense the information encoded within the particular type of hologram used as a QAM. Preferably, however, a universal hologram reader is employed so that various hologram QAMs on the same or different components, supplies and kits may be read by the same reader.

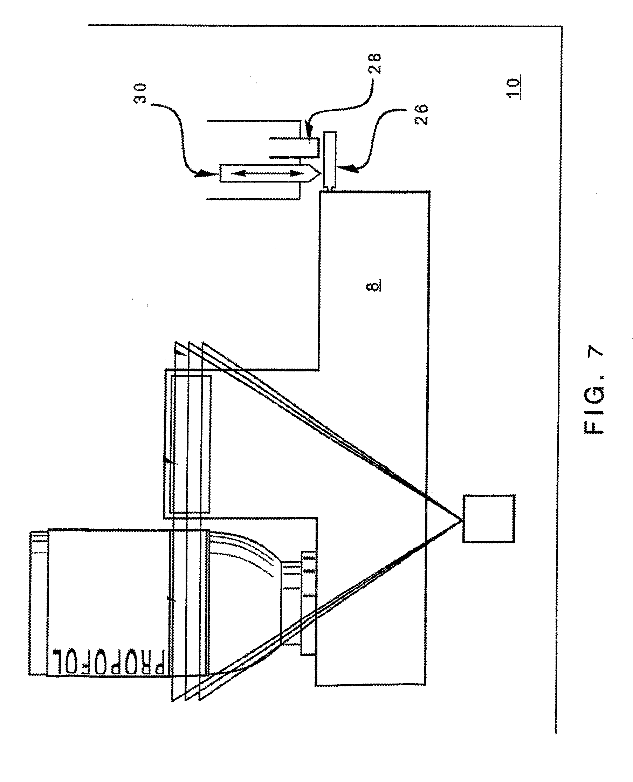

**FIG. 7** shows reuse indicator or fin **26** on cassette **8** that is sensed by reuse detector **28** of administration device or system **10**. Upon cassette 8 being inserted into place on device or system **10**, use indicating mechanism **30** of device or system **10** may move towards indicator **26** in order to break, puncture, or otherwise modify it. Mechanism **30** may also remain in a fixed location such that it breaks, punctures, or otherwise modifies indicator **26** upon cassette **8** being removed from device or system **10.** If cassette **8** is inserted in its proper position with administration device or system **10** and reuse detector **28** senses an intact or unmodified indicator 26, detector **28** will send a signal of such discovery to the drug flow controller of device or system **10.** The drug flow controller may not allow drug administration unless the signal indicates that indicator **26** is intact.

If a used and tainted cassette **8** with a modified indicator **26** is again placed into position with administration device or system **10**, reuse detector **28** of device or system **10** will sense the change and will not send a signal to the drug flow controller that indicates that the indicator is intact, i.e., that the cassette has never been used. This embodiment of the present invention ensures the safety and quality of automated drug administration to a patient by providing a means by which used and tainted cassettes are detected and rejected from further use. Alternatively, as described above with reference to **FIG. 1**, a unique serial number, similar to the unique serial number for the drug container, may also be encoded in the drug cassette QAM to prevent re-use of tainted cassettes and its attendant risk of cross-contamination.

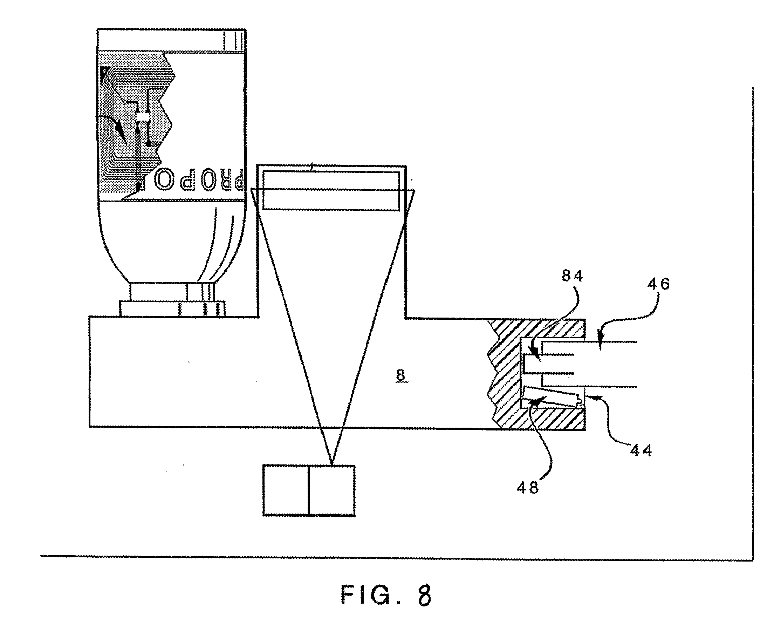

**FIG. 8** also shows an alternative embodiment of cassette reuse quality control mechanism **46** of the present invention. Reuse indicator **48** is shown in a cut-away view as having been punctured by use indicating mechanism 46 thereby leaving hole **44** which is detectable by sensor **84** provided with the mechanism **46.** Upon a cassette **8** being placed onto drug administration device or system **10**, sensor **84** on mechanism **46** can detect whether indicator **48** is in an unpunched, i.e., unused, state or whether hole **44** exists.

## Claims

1. An integrated drug delivery system, said system comprising:
a patient health monitor device adapted so as to be coupled to a patient and generate a signal reflecting at least one physiological condition of the patient;
a drug delivery controller supplying one or more drugs to the patient;
at least one medical product removably coupled to the system, said product having a quality assurance marker for storing information regarding the product, wherein the quality assurance marker comprises an identifier unique to the medical product and/or to the origin of the product;
a reader device for reading information stored on the quality assurance marker of said medical product; and
an electronic controller accessing a safety data set reflecting parameters of at least one monitored patient physiological condition and reflecting parameters of the medical product and interconnected between the patient health monitor, the drug delivery controller, and the reader device, wherein said electronic controller receives said signals from said patient health monitor device and the information stored on the quality assurance marker from the reader, and compares said information with the parameters so as to manage the application of the drugs.

2. The integrated drug delivery system of claim 1, wherein said medical product is selected from the group consisting of a drug administration drug container, a drug administration cassette, and a drug administration kit.

3. The integrated drug delivery system of claim 1, wherein said medical product is a drug container, and wherein said information further comprises information relating to properties of the drug container and the drug provided therein.

4. The integrated drug delivery system of claim 1, wherein said information further comprises information selected from the group consisting of the concentration of the drug provided in the container, the drug mix, identification of the manufacturer of the drug and/or the container, an expiration date of the drug and/or the container, the dimensions of the container, the nominal drug volume when the container is full of drug, a network address.

5. The integrated drug delivery system of claim 1, wherein said medical product is a drug cassette, and wherein said information further comprises information relating to properties of the drug cassette.

6. The integrated drug delivery system according to claim 5, wherein said information further comprises information selected from the group consisting of identification of the manufacturer of the cassette, a product ID for the cassette, a lot number for the cassette, an expiration date for the cassette, the internal or dead space volume of drug flow channels associated with the cassette, and a value reflecting the flow resistance of drug flow channels associated with the cassette.

7. The integrated drug delivery system according to claim 5, wherein a drug container having a quality assurance module storing information relating to the container is coupled to the cassette, and wherein the quality assurance module of the cassette is positioned such that quality assurance modules of both the container and the cassette are within a common scan field of a reader device which senses the information stored on the modules.

8. The integrated drug delivery system of claim 1, wherein said drug delivery system is used for sedation and analgesia, deep sedation, and/or general anesthesia.

9. The integrated drug delivery system of claim 1, wherein the information includes identifying information of the medical product, and wherein the managed delivery of drugs by the electronic controller comprises the acceptance or prevention of the use of the medical product based on the comparison of said identifying information to the information stored in the safety data set.

10. The integrated drug delivery system of claim 1, wherein the system further comprises a mechanism for altering the medical product in a particular way when the product is coupled with the system and a reuse detector which generates a signal based on the existence or absence of the particular alteration in the medical product when it is coupled with the system, and wherein the electronic controller is further interconnected with the reuse detector and receives the signal generated by the detector, wherein the managed delivery of drugs by the electronic controller comprises the acceptance or prevention of the use of the medical product based on the signal generated by the detector.

11. The integrated drug delivery system of claim 1, wherein the system further comprises a second medical product removably coupled to the system, said second product having a second quality assurance marker for storing information regarding the second product.

12. The integrated drug delivery system of claim 11, wherein the reader is capable of sensing information stored on both quality assurance markers.

13. The integrated drug delivery system of claim 1, wherein the memory device further stores the identifier of the medical product once the product has been used with the system.

14. A drug delivery apparatus, said apparatus comprising:
an electronic drug delivery controller supplying one or more drugs to the patient; and at least one medical product removably coupled to the apparatus, said product having a quality assurance marker for storing information regarding the product, wherein the quality assurance marker comprises an identifier unique to the medical product.

15. An integrated drug delivery system for enabling a non-anesthetist to safely deliver a sedative and/or analgesic drug to a patient undergoing a medical and/or surgical procedure, said system comprising:
a patient health monitor device adapted so as to be coupled to a patient and generate a signal reflecting at least one physiological condition of the patient;
a drug delivery controller supplying one or more sedative and/or analgesic drugs to the patient for providing sedation and/or pain relief to said patient during a medical procedure;
at least one medical product removably coupled to the system, said product having a quality assurance marker containing information regarding the product to provide assurance to said non-anesthetist that said product is compatible with said drug delivery controller.
